# EUROPEAN PATENT APPLICATION

(11) **EP 4 529 840 A1**
(43) Date of publication of application: **02.04.2025**
(21) Application number: 23827266.0
(22) Date of filing: 22.06.2023
(51) Int. Cl.: A61B 5/151

(54) **INFANT LANCET ASSEMBLY**

(30) Priority: 23.06.2022 JP 2022101365
(71) Applicant: Asahi Polyslider Company, Limited, Osaka 530-0005 (JP)
(72) Inventor: HIRATA, Junya, Maniwa-shi, Okayama 719-3226 (JP)
(74) Representative: Murgitroyd & Company
(86) International application number: PCT/JP2023/023155
(87) International publication number: WO 2023/249084

(57) **Abstract**

An embodiment of the present disclosure provides an infant lancet assembly comprising: a housing; and a lancet, a movable member, and a compression coil spring capable of biasing the movable member in a predetermined first direction, each of which is accommodated in the housing, wherein the housing comprises: a housing first portion having a longitudinal side extending in the first direction; and a housing second portion having a longitudinal side extending in a second direction from a side of the housing first portion, the second direction being perpendicular to the first direction, the lancet comprises a lancet body and a blade attached to the lancet body, the lancet body comprises: a body first portion provided to be movable together with the movable member; and a body second portion that is engageable with the housing second portion in a concavo-convex manner in the first direction, and the movable member comprises: a movable member first portion that is movable in the first direction and is engageable with the housing first portion in a concavo-convex manner in the second direction; and a movable member second portion on which the body first portion is disposed in an axially rotatable manner.

## Description

### TECHNICAL FIELD

The present disclosure relates to an infant lancet assembly.

### BACKGROUND ART

An infant lancet assembly is used to collect a small amount of blood sample from an infant. This blood sample can be collected mainly by incising an infant's heel. The infant lancet assembly may be referred to as a heel stick since it is used for incising an infant's hell.

### PRIOR ART DOCUMENT

### PATENT DOCUMENT

Patent Document 1: JP-A-2011-529376

### SUMMARY OF THE INVENTION

### PROBLEMS TO BE SOLVED BY THE INVENTION

Here, the present inventors have recognized that conventional infant lancet assemblies have a relatively complicated structure, and it is difficult to assemble each of the components. The difficulty in assembling each component can lead to a decrease in efficiency of production of infant lancet assemblies. Therefore, there has been a demand for an infant lancet assembly having a simple structure.

In view of this point, the present disclosure aims to provide an infant lancet assembly having a simple structure.

### SOLUTIONS TO THE PROBLEMS

In order to solve the above problems, an embodiment of the present disclosure provides
an infant lancet assembly comprising: a housing; and a lancet, a movable member, and a compression coil spring capable of biasing the movable member in a predetermined first direction, each of which is accommodated in the housing, wherein
the housing comprises: a housing first portion having a longitudinal side extending in the first direction; and a housing second portion having a longitudinal side extending in a second direction from a side of the housing first portion, the second direction being perpendicular to the first direction,
the lancet comprises a lancet body and a blade attached to the lancet body,
the lancet body comprises: a body first portion provided to be movable together with the movable member; and a body second portion that is engageable with the housing second portion in a concavo-convex manner in the first direction, and
the movable member comprises: a movable member first portion that is movable in the first direction and is engageable with the housing first portion in a concavo-convex manner in the second direction; and a movable member second portion on which the body first portion is disposed in an axially rotatable manner.

### EFFECTS OF THE INVENTION

According to the present disclosure, it is possible to provide an infant lancet assembly having a simple structure.

### BRIEF DESCRIPTION OF THE DRAWINGS

[Fig. 1] Fig. 1 is a perspective view schematically showing an infant lancet assembly according to an embodiment of the present disclosure in a state before a use of the infant lancet assembly.
[Fig. 2] Fig. 2 is a partially exploded perspective view schematically showing the infant lancet assembly according to the embodiment of the present disclosure in a state before a use of the infant lancet assembly.
[Fig. 3] Fig. 3 is a perspective view schematically showing a first sub housing of a housing that is a component of the infant lancet assembly according to the embodiment of the present disclosure.
[Fig. 4] Fig. 4 is a perspective view schematically showing a second sub housing of the housing that is a component of the infant lancet assembly according to the embodiment of the present disclosure.
[Fig. 5] Fig. 5 is a perspective view schematically showing a lancet that is a component of the infant lancet assembly according to the embodiment of the present disclosure.
[Fig. 6] Fig. 6 is a perspective view schematically showing the lancet as viewed from the opposite side from that in Fig. 5.
[Fig. 7] Fig. 7 is a perspective view schematically showing a movable member that is a component of the infant lancet assembly according to the embodiment of the present disclosure.
[Fig. 8] Fig. 8 is a perspective view schematically showing the movable member as viewed from a different side from that in Fig. 7.
[Fig. 9] Fig. 9 is a partially exploded cross-sectional view (corresponding to the view in the middle in Fig. 2) schematically showing the infant lancet assembly according to the embodiment of the present disclosure in a state before use-start.
[Fig. 10] Fig. 10 is a partially exploded cross-sectional view schematically showing the infant lancet assembly according to the embodiment of the present disclosure in the state upon the start of use (when the trigger is pushed in).
[Fig. 11] Fig. 11 is a partially exploded cross-sectional view schematically showing the infant lancet assembly according to the embodiment of the present disclosure in a process of use.
[Fig. 12] Fig. 12 is a partially exploded cross-sectional view schematically showing the infant lancet assembly according to the embodiment of the present disclosure in the state upon the end of use.

### DETAILED DESCRIPTION

The present inventors have intensively studied an infant lancet assembly having a simple structure, and, as a result, are to provide an infant lancet assembly according to an embodiment of the present disclosure.

To provide an infant lancet assembly having a simple structure, the present disclosure is based on the idea of controlling movement of a lancet with the use of a combination of a housing having a characteristic structure, and a compression coil spring that can extend in one direction, a movable member that can move in one direction when being biased by the compression coil spring, and a lancet whose predetermined portion is connected to the movable member, each of which is accommodated in the housing.

### Configuration of a Lancet Assembly

In the description below, a principal configuration of a lancet assembly 1000 according to an embodiment of the present disclosure based on the above technical idea is explained, with reference to the drawings illustrating a state before an operation of the lancet assembly.

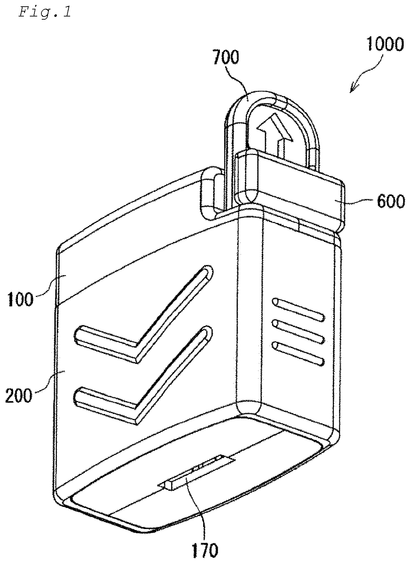

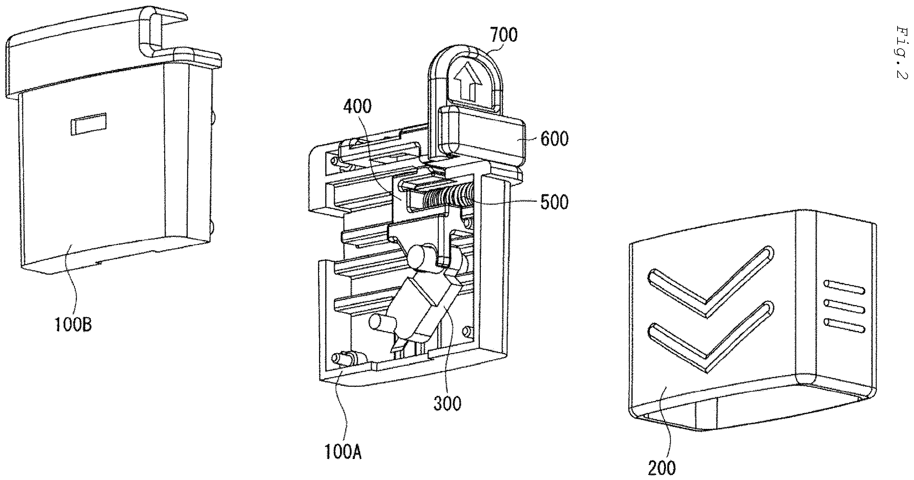

Fig. 1 is a perspective view schematically showing an infant lancet assembly according to an embodiment of the present disclosure in a state before a use of the infant lancet assembly. Fig. 2 is a partially exploded perspective view schematically showing the infant lancet assembly according to the embodiment of the present disclosure in a state before a use of the infant lancet assembly.

The lancet assembly 1000 according to the embodiment of the present disclosure includes the following principal components: a housing 100; a housing cover 200; a lancet 300, a movable member 400, and a compression coil spring 500 capable of biasing the movable member 400, each of which is placed in the housing 100; a trigger 600; and a stopper 700 that restricts movement of the trigger 600 before use (see Figs. 1 and 2). The "compression coil spring" in the present disclosure refers to a spring in a compressed state before use, and refers to a spring in which a stretched state is maintained when the compressed state is released and is switched to the stretched state. That is, in the present disclosure, a configuration in which the "compression coil spring" returns from the stretched state to the compressed state is not assumed. Note that, in the present specification, a combination of the housing 100 with the housing cover 200 covering the housing 100 is collectively referred to as a combined housing.

In the description below, the housing 100, the lancet 300, and the movable member 400 among the above components are mainly explained in detail.

### (Housing 100)

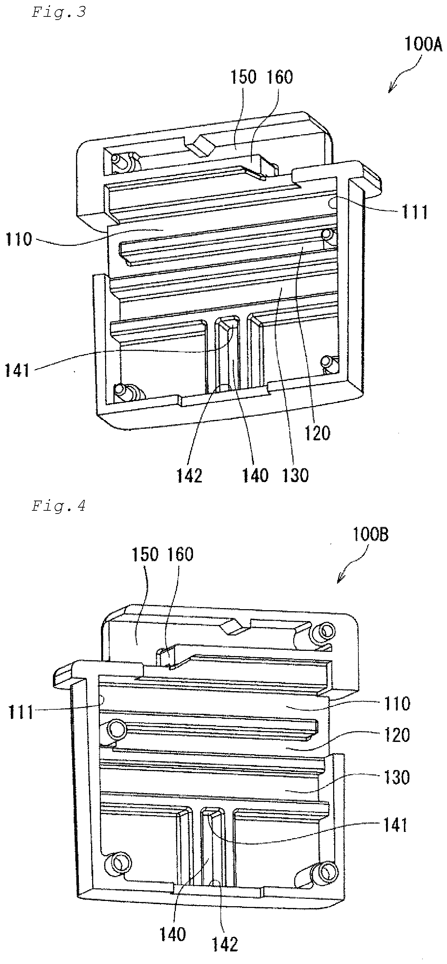

Fig. 3 is a perspective view schematically showing a first sub housing of the housing that is a component of the infant lancet assembly according to the embodiment of the present disclosure. Fig. 4 is a perspective view schematically showing a second sub housing of the housing that is a component of the infant lancet assembly according to the embodiment of the present disclosure.

The housing 100 may include a first sub housing 100A and a second sub housing 100B that can be connected to each other, for example (see Figs. 3 and 4). As an example, the interconnection of both sub housings can be realized by making the principal surface side of the first sub housing 100A and the principal surface side of the second sub housing 100B face each other, and fitting a protrusion locally disposed on an inner surface of one sub housing into a recess locally disposed on an inner surface of the other sub housing. The two sub housings can constitute the single housing 100. In a state where the two sub housings interconnected, the housing 100 can have an opening portion 170 through which a blade of the lancet 300 described later can protrude outward (see Fig. 1). In the aspect illustrated in Figs. 3 and 4, the insides of both the sub housings may have a concave-convex shape. However, the present embodiment is not limited to this, and the inside of one sub housing may have an uneven shape while the inside of the other sub housing has a planar shape.

In the description below, the configuration in the first sub housing 100A of the housing 100 is mainly explained. As illustrated in Fig. 3, (the first sub housing 100A of) the housing 100 includes at least one housing first portion 110, 120 having a longitudinal side extending in a first direction (corresponding to the lateral direction) and a housing second portion 140 having a longitudinal side extending from the housing first portion side in a second direction (corresponding to the vertical direction) perpendicular to the first direction. Note that the second sub housing 100B may also include a housing first portion having a longitudinal side extending in the first direction (corresponding to the lateral direction) and a housing second portion having a longitudinal side extending in the second direction (corresponding to the vertical direction). In a state where the two sub housings interconnected, respective housing first portions in both sub housings 100A, 100B are disposed to be face each other and respective housing second portions in both sub housings 100A and 100B can be arranged to face each other, at least.

The housing first portion 110, 120 can be engaged with a later-described movable member first portion in a concavo-convex manner in the second direction (corresponding to the vertical direction). That is, on the assumption that the longitudinal side of the housing first portion 110, 120 extends in the first direction, the short side of the housing first portion 110, 120 can be engaged with the movable member first portion as a whole in a concavo-convex manner in the second direction (corresponding to the vertical direction). Note that the housing first portion 110, 120 can be engaged with the later-described movable member first portion in a concavo-convex manner in the second direction (corresponding to the vertical direction).

The term "engaged in a concavo-convex manner" as used in the present specification refers to a state in which one member (corresponding to the housing first portion or the like) has a concave shape, the other member (corresponding to the movable member first portion or the like) has a convex shape, and the two members can be engaged with each other by virtue of their shapes, or a state in which one member (corresponding to the housing first portion or the like) has a convex shape, the other member (corresponding to the movable member first portion or the like) has a concave shape, and the two members can be engaged with each other by virtue of their shapes. With the housing first portion and the movable member first portion being taken as an example, "concavo-convex engagement" refers to a state in which the housing first portion and the movable member first portion can be engaged with each other so that the first portion of the movable member can move relatively in the first direction (corresponding to the lateral direction) but does not move relatively in the second direction (corresponding to the vertical direction) because of the shapes of both portions. Note that, in the present embodiment, an aspect in which the two housing first portions 110 and 120 are disposed apart from each other is described as an example. However, one or more housing first portions that can be engaged with the later-described movable member first portion in a concavo-convex manner may be provided without being limited to this example.

Specifically, between the housing first portion 110, 120 and the movable member first portion, the protruding portion of one of the housing first portion and the movable member first portion can come into contact with the inner surface forming the recessed portion of the other. As a result, movement of the movable member first portion in the second direction is restricted, and thus, movement of the movable member in the first direction (corresponding to the lateral direction) can be easily controlled as a whole.

In the aspect illustrated in the drawings, two housing first portions 110 and 120 that are spaced apart from each other are provided. In this case, one end 111 (corresponding to a side surface of the housing) of the housing first portion 110 located at the uppermost position can be in contact with one side 510 of the compression coil spring 500, for example. The other side 520 of the compression coil spring 500 can be connected to the movable member 400 described later, or, more specifically, to a protruding portion 440 of the movable member 400.

Note that the number of the housing first portions may be at least one, as long as the housing first portion can control movement of the movable member in the first direction (corresponding to the lateral direction). Further, to control movement of the movable member in the first direction (corresponding to the lateral direction) in a more preferred manner, the number of the housing first portions can be two or more.

Note that, to improve such movement control on the movable member, the width dimension of the housing first portion 110, 120 in the second direction (corresponding to the vertical direction) is preferably a dimension with which the movable member first portion can slide in the first direction (corresponding to the lateral direction) with respect to the housing first portion 110, 120.

The housing second portion 140 has a longitudinal side that has a first end 141 proximal to the housing first portion 110, 120, and a second end 142 distal to the housing first portion. Further, the housing second portion 140 can be engaged in a concavo-convex manner with a body second portion of a lancet body that is a component of the later-described lancet in the first direction (corresponding to the lateral direction).

That is, on the assumption that the longitudinal side of the housing second portion 140 extends in the second direction, the short side of the housing second portion 140 can be engaged with the body second portion of the lancet body as a whole in a concavo-convex manner in the first direction (corresponding to the lateral direction). Specifically, between the housing second portion 140 and the body second portion, the protruding portion of one of the housing second portion 140 and the body second portion can come into contact with the inner surface forming the recessed portion of the other. With this arrangement, movement of the body second portion in the first direction is restricted, and thus, movement of the body second portion in the second direction can be easily controlled as a whole.

Note that, to improve the movement control on the body second portion, the width dimension of the housing second portion 140 in the first direction is preferably a dimension with which the body second portion can slide in the second direction with respect to the housing second portion 140. As a result, the movement control on the body second portion is improved. Thus, as will be described later, when a blade of the lancet 300 is made to protrude outward with movement of the body second portion in the second direction in the process of use, the operation of the blade can be stabilized.

In the embodiment of the present invention, the housing first portion 110, 120 extends in the first direction (corresponding to the lateral direction), and the housing second portion 140 extends in the second direction (corresponding to the vertical direction) from the housing first portion side. As such an extending form is adopted, the housing first portion 110, 120 and the housing second portion 140 are arranged so as to form a T-shape as a whole. Note that the "T-shaped arrangement of the housing first portion and the housing second portion" in the present specification includes not only an arrangement in which the housing first portion and the housing second portion are physically in contact with each other, but also an arrangement in which the housing first portion and the housing second portion are separated from each other by a predetermined distance and form a substantially T-shape as a whole.

The housing 100 (or the first sub housing 100A thereof) may include a housing third portion 130 that can be engaged in a concavo-convex manner with a body first portion 311 of the later-described lancet in the second direction, and a housing fourth portion 150 that can accommodate the trigger 600. The housing third portion 130 and the housing fourth portion 150 may have an arrangement in which the two portions 130 and 150 extend in substantially the same direction while maintaining a predetermined distance from the housing first portions. Also, the housing 100 includes a locking portion 160 capable of locking a flexible portion 450 of the later-described movable member 400.

In a case where the housing third portion 130 is provided, the housing third portion 130 can be engaged with the body first portion 311 of the lancet in a concavo-convex manner, and thus, movement of the body first portion 311 of the lancet in the second direction can be restricted. With this arrangement, movement of the body first portion 311 of the lancet in the first direction (corresponding to the lateral direction) can be easily controlled as a whole.

Further, in the mode illustrated in the drawings, the housing first portion 110, the housing second portion 140, and the housing third portion 130 can be in a recessed form. In this case, the later-described mating components (the movable member and the lancet) compatible with an engagement in a concavo-convex manner may locally have a protruding form. Note that the present embodiment is not limited to this, and the housing first to third portions may each have a protruding form. In this case, the mating components (the movable member and the lancet) compatible with an engagement in a concavo-convex manner locally have a recessed form.

### (Lancet 300)

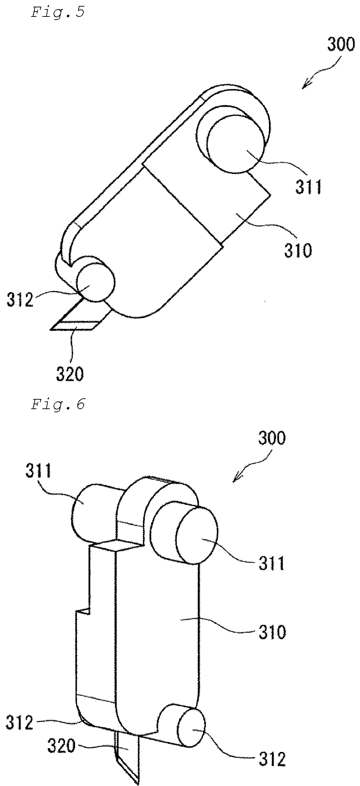

Fig. 5 is a perspective view schematically showing a lancet that is a component of the infant lancet assembly according to the embodiment of the present disclosure. Fig. 6 is a perspective view schematically showing the lancet as viewed from the opposite side from that in Fig. 5.

The lancet 300 includes a lancet body 310 and a blade 320 attached to the lancet body 310. The lancet body 310 includes the body first portion 311 disposed on one end side of the longitudinal side, and a body second portion 312 disposed on the other end side.

The body first portion 311 can move together with the movable member 400, and is connected to the movable member 400 in an axially rotatable manner. In the embodiment, the position of the connecting portion of the axially rotatable body first portion 311 with respect to the movable member 400 can be maintained. The body first portion 311 can be engaged in a concavo-convex manner with the above-described housing third portion 130 in the second direction. With this arrangement, movement of the body first portion 311 of the lancet in the second direction can be adequately restricted, and thus, movement of the body first portion 311 in the first direction can be easily controlled.

Also, the body second portion 312 can be engaged in a concavo-convex manner with the housing second portion 140 in the first direction. With this arrangement, movement of the body second portion in the first direction is restricted, and thus, movement of the body second portion in the second direction can be easily controlled.

Before the movable member 400 starts moving in the first direction, the body second portion 312 is configured to be slidable between the first end 141 and the second end 142 of the housing second portion 140. That is, the body second portion 312 of the lancet body 310 can be disposed directly with respect to its own housing 100. In this case, the blade 320 is configured so that it can be accommodated in the housing 100.

Further, during the movement after the start of movement of the movable member 400 in the first direction, the body second portion 312 can be positioned on the side of the second end 142 of the housing second portion 140 of the housing 100, and, at this point of time, the blade 320 can protrude outward through the opening portion of the housing 100. Note that, to keep the degree of outward protrusion of the blade 320 at a constant level, the body second portion 312 comes into contact with the second end 142 of the housing second portion 140, and thus, movement of the body second portion 312 to a more distal side than the second end 142 can be restricted.

As described above, in a case where the housing 100 includes the first sub housing 100A and the second sub housing 100B, it is preferable that the body first portion 311 can be disposed in the same row or on the same axis on each of the two facing principal surfaces of the lancet body 310.

With such an arrangement, it is possible to ensure two concavo-convex engagement points between the body first portion 311 and the housing third portion 130 in the second direction. Thus, movement of the body first portion 311 of the lancet in the second direction can be restricted in a more preferred manner. As a result, movement of the body first portion 311 of the lancet in the first direction (corresponding to the lateral direction) can be controlled in a more preferred manner as a whole.

Likewise, in a case where the housing 100 includes the sub housing 100A and the second sub housing 100B, it is preferable that the body second portion 312 can be disposed in the same row or on the same axis on each of the two facing principal surfaces of the lancet body 310.

With such an arrangement, it is possible to ensure two concavo-convex engagement points between the body second portion 312 and the housing second portion 140 in the first direction. Thus, movement of the body second portion 312 of the lancet in the first direction can be restricted in a more preferred manner. As a result, movement of the body second portion 312 of the lancet in the second direction (corresponding to the vertical direction) can be easily controlled in a more preferred manner as a whole.

### (Movable Member 400)

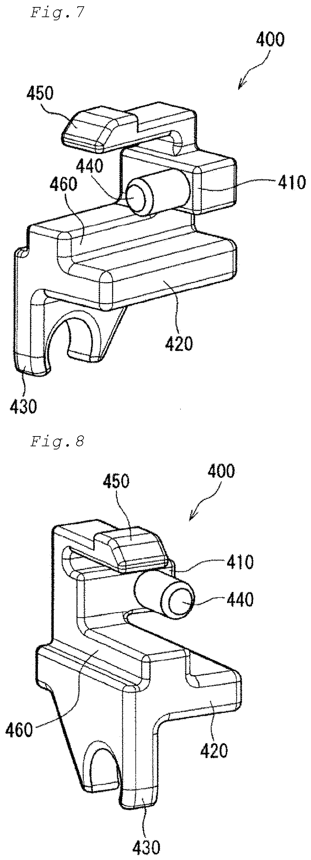

Fig. 7 is a perspective view schematically showing the movable member that is a component of the infant lancet assembly according to the embodiment of the present disclosure. Fig. 8 is a perspective view schematically showing the movable member as viewed from a different side from that in Fig. 7.

The movable member 400 can be configured to be biased in the first direction (corresponding to the lateral direction) by the compression coil spring 500. The movable member 400 includes a protruding portion 440 that can be connected to the compression coil spring 500. The protruding portion 440 can be disposed at any position on the movable member 400, as long as the compression coil spring 500 can bias the movable member 400.

Also, the movable member 400 includes at least one movable member first portion 410 that can be engaged with the housing first portion in a concavo-convex manner, a movable member second portion 430 on which the body first portion 311 of the lancet 300 can be placed in an axially rotatable manner, and the flexible portion 450 that can be locked with a predetermined portion (corresponding to the locking portion 160) of the housing 100 before use. In a case where one side of the flexible portion 450 is continuous with the movable member first portion 410, for example, the other side is in a free state to have flexibility.

Further, the flexible portion 450 is disposed at a position at which the flexible portion 450 can come into contact with the trigger 600 during use. Specifically, the stopper 700 that restricts movement of the trigger 600 is removed upon the start of use, so that the trigger 600 can be manually pushed in and moved. As a result, when coming into contact with the trigger 600, the flexible portion 450 can be bent in a direction in which the locking with the predetermined portion (corresponding to the locking portion 160) of the housing 100 can be released by virtue of its properties. As a result, the locking between the two can be released.

Furthermore, in a case where the two housing first portions 110 and 120 spaced apart from each other are provided as described above, two movable member first portions 410 and 420 spaced apart from each other can be provided as to be engageable with each other in a concavo-convex manner in the aspect illustrated in the drawings (see Figs. 7 and 8). In this case, a movable member third portion 460 (corresponding to an intervening portion or a first-direction extending portion) that can slide with the housing 100 and extends in the first direction may be provided between the two movable member first portions 410 and 420. Further, when viewed in a cross-section, the movable member second portion 430 can be disposed in a direction perpendicular to the longitudinal-side extending direction of the movable member first portion 410, 420, for example.

### Operation of the Lancet Assembly

An operation of the lancet assembly 1000 according to the embodiment of the present disclosure based on the above configuration is now described.

### (Before Start of Use)

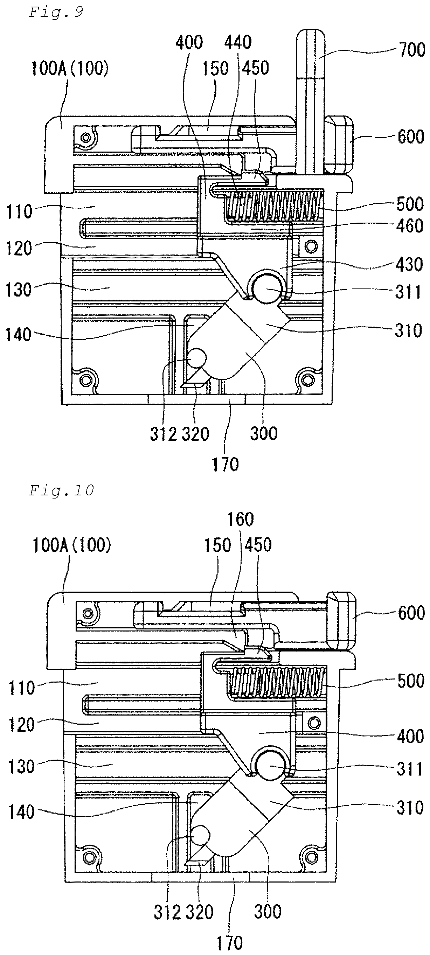

Fig. 9 is a partially exploded cross-sectional view (corresponding to the view in the middle in Fig. 2) schematically showing the infant lancet assembly according to the embodiment of the present disclosure in a state before a use of the infant lancet assembly.

Before the start of use, movement of the trigger 600 is restricted by the stopper 700. Accordingly, the flexible portion 450 of the movable member 400 is locked with the locking portion 160 of the housing 100. In this state, the movable member 400 connected to the coil spring 500 in a compressed state on one side (the right-side end in Fig. 9) of the housing 100 maintains a resting state. In such a resting state, the body second portion of the lancet body 310, which is a component of the lancet 300, is located between the first end 141 and the second end 142 of the housing second portion 140, and the blade 320 is located in the housing 100.

### (Upon the Start of Use)

Fig. 10 is a partially exploded cross-sectional view schematically showing the infant lancet assembly according to the embodiment of the present disclosure in the state upon the start of use (when the trigger is pushed in).

Upon the start of use, the stopper is removed so that the trigger 600 can be pushed into the housing 100 (see Fig. 10). After that, when the trigger 600 is pushed in by the user, the trigger 600 comes into contact with the flexible portion 450 of the movable member 400, and, at the time of this contact, the flexible portion 450 bends in a direction in which the locking with the locking portion 160 of the housing 100 can be released. As a result, the locking between the two can be released.

### (During Use)

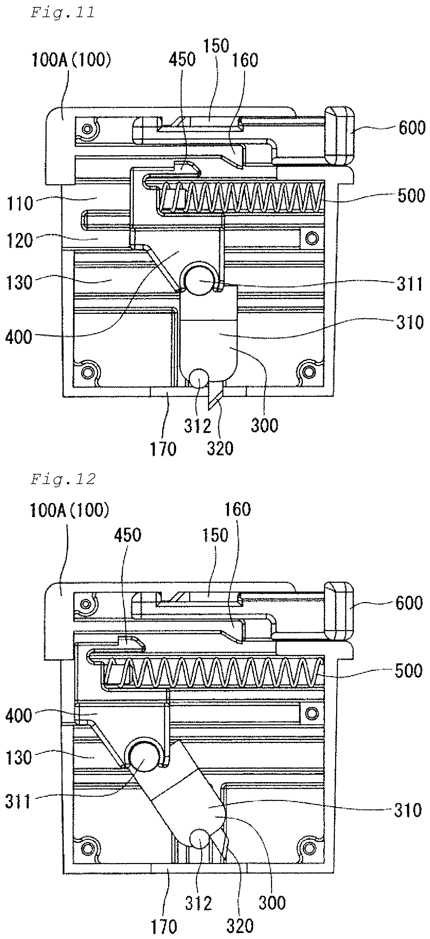

Fig. 11 is a partially exploded cross-sectional view schematically showing the infant lancet assembly according to the embodiment of the present disclosure in the process of use.

When the locking between the flexible portion 450 of the movable member 400 and the locking portion 160 of the housing 100 is released, the compressed state of the compression coil spring 500 is released. By such compression release, the movable member 400 can be biased in the first direction by the compression coil spring 500. As a result, the movable member 400 can start moving in the first direction.

In the embodiment of the present disclosure, the body first portion 311 of the lancet body 310, which is a component of the lancet 300, can move together with the movable member 400 in the first direction, as described above. On the other hand, the body second portion of the lancet body 310 can be engaged in a concavo-convex manner with the housing second portion 140 in the first direction.

With such a configuration, when the movable member 400 moves in the first direction, the body first portion 311 also moves in the first direction together with the movement of the movable member 400, whereas the movement of the concavo-convexly engaged body second portion 312 in the first direction is restricted. As a result, it may be possible to provide the body first portion 311 with an axially rotating force. As the body first portion 311 moves in the first direction by the action of such an axially rotating force, the movement of the body second portion in the first direction (lateral direction) that can be engaged in a concavo-convex manner with the housing second portion 140 is restricted, while a force of moving the body second portion in the second direction (vertical direction) is applied.

As described above, before the movable member 400 starts moving in the first direction, the body second portion 312 of the lancet body 310 is configured to be located between the first end 141 and the second end 142 of the housing second portion 140. After that, in the process of movement of the movable member 400 in the first direction, a force of moving the body second portion in the second direction (vertical direction) is applied as the body first portion 311 moves in the first direction. As a result, the body second portion 312 can move from the side of the first end 141 toward the side of the second end 142 of the housing second portion 140.

Further, in the process of movement of the movable member 400 in the first direction, when the body second portion 312 is configured to be located on the side of the second end 142 of the housing second portion 140, the blade 320 can be configured to protrude outward through the opening portion 170 of the housing 100, as described above. As a result, the blade 320 can protrude outward from the inside of the housing 100 through the opening portion 170 of the housing 100 in the middle of the movement of the movable member 400 in the first direction (see Fig. 11).

Note that, as described above, if the housing second portion 140 in the first direction has a dimension with which the body second portion 312 can slide in the second direction with respect to the housing second portion 140, the movement control on the body second portion 312 can be enhanced. As a result, when the blade 320 is made to protrude outward as the body second portion 312 moves in the second direction in the process of use, the operation of the blade 320 can be stabilized.

### (At the End of Use)

Fig. 12 is a partially exploded cross-sectional view schematically showing the infant lancet assembly according to the embodiment of the present disclosure in the state at the end of use.

In the embodiment of the present disclosure, the body first portion 311 of the lancet body 310, which is a component of the lancet 300, can move together with the movable member 400, as described above. On the other hand, the body second portion of the lancet body 310 can be engaged with the housing second portion 140 in a concavo-convex manner.

As such a configuration is adopted, when the movable member 400 further moves in the first direction until coming into contact with the other side (the left-side end in Fig. 12) of the housing 100, the body first portion 311 also moves in the first direction together with the further movement of the movable member 400, whereas the movement of the concavo-convexly engaged body second portion 312 in the first direction is restricted.

As a result, it may be possible to further provide the body first portion 311 with an axially rotating force. As the body first portion 311 further moves in the first direction by virtue of the further provision of the axially rotating force, the body second portion 312 can move from the side of the second end 142 toward the side of the first end 141 of the housing second portion 140. As a result, the body second portion 312 of the lancet body 310 can be located between the first end 141 and the second end 142 of the housing second portion 140, and the blade 320 can be accommodated in the housing 100 as in the state before the start of use.

Further, as described above, in the present disclosure, it is assumed that the compression coil spring 500 is maintained in a stretched state when the compression coil spring 500 is released from a compressed state and is stretched. Therefore, the movable member 400 connected to the compression coil spring 500 does not return to the one side (the right-side end in Fig. 9) of the housing 100 after coming into contact with the other side (the left-side end in Fig. 12) of the housing cover 200 and/or the housing 100 in the first direction, which is the combined housing. Because of this, the movable member 400 does not further move, and accordingly, and thus it does not cause a force for moving again toward the second end 142 of the housing second portion 140 for the body second portion 312 of the lancet 300 connected to the movable member 400.

Thus, the state in which the blade 320 is accommodated in the housing 100 can be maintained. As a result, after a small amount of blood sample is collected from an infant with the blade that has once come out of the housing 100, the blade 320 with blood can be suitably prevented from coming out of the housing 100 again. Thus, the safety after use of the infant lancet assembly can also be ensured in a preferred manner.

In view of the above, in the present disclosure, the compression coil spring 500 that can be stretched in one direction, the movable member 400 that can move in one direction when biased by the compression coil spring 500, and the lancet 300 whose predetermined portion is connected to the movable member 400 can be combined as a simple structure in the housing 100 having the characteristic structure as described above. In the present disclosure, movement of the blade 320 of the lancet 300 can be suitably controlled with the infant lancet assembly 1000 having such a simple structure. In this regard, the infant lancet assembly of the present disclosure has technical significance.

Although one embodiment of the present disclosure has been described so far, the embodiment is merely a typical example within the scope of application of the present disclosure. Therefore, the present disclosure is not limited to this example, and those skilled in the art will readily understand that various modifications can be made to it.

Note that the embodiment of the present invention as described above includes the following preferred aspects.

### First aspect:

An infant lancet assembly comprising: a housing; and a lancet, a movable member, and a compression coil spring capable of biasing the movable member in a predetermined first direction, each of which is accommodated in the housing, wherein
the housing comprises: a housing first portion having a longitudinal side extending in the first direction; and a housing second portion having a longitudinal side extending in a second direction from a side of the housing first portion, the second direction being perpendicular to the first direction,
the lancet comprises a lancet body and a blade attached to the lancet body,
the lancet body comprises: a body first portion provided to be movable together with the movable member; and a body second portion that is engageable with the housing second portion in a concavo-convex manner in the first direction, and
the movable member includes: a movable member first portion that is movable in the first direction and is engageable with the housing first portion in a concavo-convex manner in the second direction; and a movable member second portion on which the body first portion is disposed in an axially rotatable manner.

### Second aspect:

In the first aspect, the housing first portion and the housing second portion are arranged to form a T-shape in the infant lancet assembly.

### Third aspect:

In the first or second aspect, a width dimension of the housing second portion in the first direction is a dimension with which the body second portion is slidable in the second direction with respect to the housing second portion in the infant lancet assembly.

### Fourth aspect:

In any of the first to third aspects, a width dimension of the housing first portion in the second direction is a dimension with which the movable member first portion is slidable in the first direction with respect to the housing first portion in the infant lancet assembly.

### Fifth aspect:

In any of the first to fourth aspects, one of the movable member first portion or the housing first portion is a protruding portion, and the other is a recessed portion in the infant lancet assembly.

### Sixth aspect:

In any of the first to fifth aspects, one of the body second portion or the housing second portion is a protruding portion, and the other is a recessed portion in the infant lancet assembly.

### Seventh aspect:

In any of the first to sixth aspects, when the movable member moves in the first direction, the body first portion is movable in the first direction together with the movable member, whereas movement of the body second portion in the first direction is restricted by the housing second portion in the infant lancet assembly.

### Eighth aspect:

In the seventh aspect dependent on the sixth aspect, between the body second portion and the housing second portion, the protruding portion of one of the body second portion or the housing second portion is capable of coming into contact with an inner surface forming the recessed portion of the other one of the body second portion and the housing second portion, which enables movement of the body second portion in the first direction to be restricted in the infant lancet assembly.

### Ninth aspect:

In any of the first to eighth aspects, when the movable member moves in the first direction, movement of the movable member first portion in the second direction is restricted by the housing first portion in the infant lancet assembly.

### Tenth aspect:

In the ninth aspect dependent on the fifth aspect, between the movable member first portion and the housing first portion, the protruding portion of one of the movable member first portion or the housing first portion is capable of coming into contact with an inner surface forming the recessed portion of the other one of the movable member first portion and the housing first portion, which enables movement of the movable member first portion in the second direction to be restricted in the infant lancet assembly.

### Eleventh aspect:

In the seventh or eighth aspect, movement of the body second portion in the first direction is restricted, to make the body first portion axially rotatable with respect to the movable member in the infant lancet assembly.

### Twelfth aspect:

In any of the first to eleventh aspects, when the movable member moves in the first direction, the body first portion is movable in the first direction together with the movable member, and the body second portion is movable in the second direction in the infant lancet assembly.

### Thirteenth aspect:

In any of the first to twelfth aspects, the longitudinal side of the housing second portion comprises a first end proximal to the housing first portion and a second end distal to the housing first portion, and the body second portion is located between the first end and the second end of the housing second portion before the movable member starts moving in the first direction in the infant lancet assembly.

### Fourteenth aspect:

In the thirteenth aspect, when the movable member moves in the first direction, the body second portion moves from a side of the first end to a side of the second end, and then moves from the second end side to the first end side in the infant lancet assembly.

### Fifteenth aspect:

In the thirteenth or fourteenth aspect, the housing comprises an opening portion through which the blade is able to protrude, and, when the body second portion is located on a side of the second end, the blade is able to protrude outward through the opening portion of the housing in the infant lancet assembly.

### Sixteenth aspect:

In any of the thirteenth to fifteenth aspects, the blade is accommodatable in the housing when the body second portion is located between the first end and the second end of the longitudinal side of the housing second portion in the infant lancet assembly.

### Seventeenth aspect:

In any of the first to sixteenth aspects, the housing comprises two or more housing first portions that have a longitudinal side extending in the first direction and are spaced apart from and facing each other, and the movable member is engageable with the two or more housing first portions in a concavo-convex manner in the infant lancet assembly.

### Eighteenth aspect:

In any of the first to seventeenth aspects, the housing comprises a first sub housing and a second sub housing, each of the first sub housing and the second sub housing comprises the housing first portion and the housing second portion, and respective housing first portions in the first sub housing and the second sub housing are disposed to be able to face each other and respective housing second portions in the first sub housing and the second sub housing are disposed to be able to face each other, at least in the infant lancet assembly.

### Nineteenth aspect:

In any of the first to eighteenth aspects, the housing further comprises a housing third portion that is engageable with the body first portion in a concavo-convex manner in the second direction in the infant lancet assembly.

### Twentieth aspect:

In the eighteenth aspect, the body first portion is disposable in a same row on each of two facing principal surfaces of the lancet body in the infant lancet assembly.

### Twenty-first aspect:

In any of the eighteenth to twentieth aspects, the body second portion is disposable in a same row on each of two facing principal surfaces of the lancet body in the infant lancet assembly.

### Twenty-second aspect:

In any of the first to twenty-first aspects, the movable member further comprises a flexible portion, the infant lancet assembly further comprises a trigger capable of coming into contact with the flexible portion, the flexible portion of the movable member and a predetermined portion of the housing can be locked with each other before use, and the trigger comes into contact with the flexible portion upon a start of use, to be able to release locking between the flexible portion and the predetermined portion of the housing.

### INDUSTRIAL APPLICABILITY

An infant lancet assembly according to an embodiment of the present disclosure is used to collect a small amount of blood sample from an infant.

### CROSS-REFERENCE TO RELATED APPLICATION

The present application claims priority based on Japanese Patent Application No. 2022-101365 (Filing Date: June 23, 2022, Title of Invention: "Infant Lancet Assembly"). The entire contents disclosed in the application are incorporated herein by this reference.

### REFERENCE SIGNS LIST

- 1000: infant lancet assembly
- 100: housing
- 100A: first sub housing
- 100B: second sub housing
- 110, 120: housing first portion
- 130: housing third portion
- 140: housing second portion
- 141: first end of housing second portion
- 142: second end of housing second portion
- 150: housing fourth portion
- 160: locking portion
- 170: opening portion
- 200: housing cover
- 300: lancet
- 310: lancet body
- 311: body first portion
- 312: body second portion
- 320: blade
- 400: movable member
- 410: movable member first portion
- 420: movable member first portion
- 430: movable member second portion
- 440: protruding portion
- 450: flexible portion
- 460: movable member third portion
- 500: compression coil spring
- 600: trigger
- 700: stopper

## Claims

1. An infant lancet assembly comprising: a housing; and a lancet, a movable member, and a compression coil spring capable of biasing the movable member in a predetermined first direction, each of which is accommodated in the housing, wherein
the housing comprises: a housing first portion having a longitudinal side extending in the first direction; and a housing second portion having a longitudinal side extending in a second direction from a side of the housing first portion, the second direction being perpendicular to the first direction,
the lancet comprises a lancet body and a blade attached to the lancet body,
the lancet body includes: a body first portion provided to be movable together with the movable member; and a body second portion that is engageable with the housing second portion in a concavo-convex manner in the first direction, and
the movable member comprises: a movable member first portion that is movable in the first direction and is engageable with the housing first portion in a concavo-convex manner in the second direction; and a movable member second portion on which the body first portion is disposed in an axially rotatable manner.

2. The infant lancet assembly according to claim 1, wherein the housing first portion and the housing second portion are arranged to form a T-shape.

3. The infant lancet assembly according to claim 1 or 2, wherein a width dimension of the housing second portion in the first direction is a dimension with which the body second portion is slidable in the second direction with respect to the housing second portion.

4. The infant lancet assembly according to claim 1 or 2, wherein a width dimension of the housing first portion in the second direction is a dimension with which the movable member first portion is slidable in the first direction with respect to the housing first portion.

5. The infant lancet assembly according to claim 1 or 2, wherein one of the movable member first portion or the housing first portion is a protruding portion, and the other is a recessed portion.

6. The infant lancet assembly according to claim 1 or 2, wherein one of the body second portion or the housing second portion is a protruding portion, and the other is a recessed portion.

7. The infant lancet assembly according to claim 6, wherein, when the movable member moves in the first direction, the body first portion is movable in the first direction together with the movable member, whereas movement of the body second portion in the first direction is restricted by the housing second portion.

8. The infant lancet assembly according to claim 7, wherein, between the body second portion and the housing second portion, the protruding portion of one of the body second portion or the housing second portion is capable of coming into contact with an inner surface forming the recessed portion of the other one of the body second portion and the housing second portion, which enables movement of the body second portion in the first direction to be restricted.

9. The infant lancet assembly according to claim 5, wherein, when the movable member moves in the first direction, movement of the movable member first portion in the second direction is restricted by the housing first portion.

10. The infant lancet assembly according to claim 9, wherein, between the movable member first portion and the housing first portion, the protruding portion of one of the movable member first portion or the housing first portion is capable of coming into contact with an inner surface forming the recessed portion of the other one of the movable member first portion and the housing first portion, which enables movement of the movable member first portion in the second direction to be restricted.

11. The infant lancet assembly according to claim 7 or 8, wherein movement of the body second portion in the first direction is restricted, to make the body first portion axially rotatable with respect to the movable member.

12. The infant lancet assembly according to claim 1 or 2, wherein, when the movable member moves in the first direction, the body first portion is movable in the first direction together with the movable member, and the body second portion is movable in the second direction.

13. The infant lancet assembly according to claim 1, wherein the longitudinal side of the housing second portion comprises a first end proximal to the housing first portion and a second end distal to the housing first portion, and the body second portion is located between the first end and the second end of the housing second portion before the movable member starts moving in the first direction.

14. The infant lancet assembly according to claim 13, wherein, when the movable member moves in the first direction, the body second portion moves from a side of the first end to a side of the second end, and subsequently moves from the second end side to the first end side.

15. The infant lancet assembly according to claim 13 or 14, wherein the housing comprises an opening portion through which the blade is able to protrude, and, when the body second portion is located on a side of the second end, the blade is able to protrude outward through the opening portion of the housing.

16. The infant lancet assembly according to claim 13 or 14, wherein the blade is accommodatable in the housing when the body second portion is located between the first end and the second end of the longitudinal side of the housing second portion.

17. The infant lancet assembly according to claim 1 or 2, wherein the housing comprises two or more housing first portions that have a longitudinal side extending in the first direction and are spaced apart from and facing each other, and the movable member is engageable with the two or more housing first portions in a concavo-convex manner.

18. The infant lancet assembly according to claim 1, wherein the housing comprises a first sub housing and a second sub housing, each of the first sub housing and the second sub housing comprises the housing first portion and the housing second portion, and respective housing first portions in the first sub housing and the second sub housing are disposed to be able to face each other and respective housing second portions in the first sub housing and the second sub housing are disposed to be able to face each other, at least.

19. The infant lancet assembly according to claim 1 or 2, wherein the housing further comprises a housing third portion that is engageable with the body first portion in a concavo-convex manner in the second direction.

20. The infant lancet assembly according to claim 18, wherein the body first portion is disposable in a same row on each of two facing principal surfaces of the lancet body.

21. The infant lancet assembly according to claim 18 or 20, wherein the body second portion is disposable in a same row on each of two facing principal surfaces of the lancet body.

22. The infant lancet assembly according to claim 1 or 2, wherein the movable member further comprises a flexible portion, the infant lancet assembly further comprises a trigger capable of coming into contact with the flexible portion, the flexible portion of the movable member and a predetermined portion of the housing are capable of being locked with each other before use, and the trigger comes into contact with the flexible portion upon a start of use, to be able to release locking between the flexible portion and the predetermined portion of the housing.
